# EUROPEAN PATENT SPECIFICATION

(11) **EP 2 460 888 B1**
(45) Date of publication and mention of the grant of the patent: **09.05.2018**
(21) Application number: 10804211.0
(22) Date of filing: 21.06.2010
(51) Int. Cl.: C12Q 1/32, C12Q 1/54, C12Q 1/58

(54) **METHOD AND KIT FOR MEASUREMENT OF DEHYDROGENASE OR SUBSTRATE FOR THE DEHYDROGENASE**
VERFAHREN UND KIT ZUM MESSEN DER DEHYDROGENASE ODER SUBSTRAT DER DEHYDROGENASE
PROCÉDÉ ET KIT DE MESURE D'UNE DÉSHYDROGÉNASE OU D'UN SUBSTRAT POUR LA DÉSHYDROGÉNASE

(30) Priority: 28.07.2009 JP 2009175379
(43) Date of publication of application: 06.06.2012
(73) Proprietor: Nitto Boseki CO., LTD., Fukushima 960-8161 (JP)
(72) Inventor: NODA Kenta, Koriyama-shi Fukushima 963-0702 (JP); SATO Yoshiro, Koriyama-shi Fukushima 963-8822 (JP); KOJIMA Ryo, Koriyama-shi Fukushima 963-0701 (JP)
(74) Representative: Hoffmann Eitle
(86) International application number: PCT/JP2010/060450
(87) International publication number: WO 2011/013464

(56) References cited:
- EP-A1- 0 639 646
- EP-A1- 1 028 165
- JP-A- 4 335 898
- JP-A- 2001 103 996
- ICHITOMO MIWA ET AL.: 'Glucose' CLINICAL TESTING vol. 22, no. 11, 1978, pages 1232 - 1261, XP008150659

## Description

### Technical Field

The present invention relates to a method for measuring a dehydrogenase or a substrate for the dehydrogenase in a sample, and use of a kit for the method.

### Background Art

In samples such as blood serum and blood plasma, dehydrogenases or substrates for the dehydrogenases are present at high concentrations. In clinical chemical diagnoses, it is general to use nicotinamide adenine dinucleotide (NAD) or nicotinamide adenine dinucleotide phosphate (NADP) as a coenzyme for the measurement of those enzymes and substrates. Some known examples of a combination of a dehydrogenase and a substrate that are used in such a method, include a lactate dehydrogenase and lactic acid, a glutamate dehydrogenase and glutamic acid, an alcohol dehydrogenase and an alcohol, a glycerol-3-phosphate dehydrogenase and glycerol-3-phosphate, and a glucose-6-phosphate dehydrogenase and glucose-6-phosphate. In this case, in order to measure any one of the members of the combinations, the other member is used as a reagent together with a coenzyme. Similarly, in order to measure a particular substance such as urea in a sample, a technique of subjecting the particular substance to an enzymatic reaction using a reagent so as to produce a particular dehydrogenase or a substrate for the dehydrogenase as an intermediate or a final product, thereby allowing the dehydrogenase or the substrate for the dehydrogenase to react with the coenzyme described above, which results in measuring the particular substance, is also widely performed.

In regard to these measurement methods, it is general to employ a technique in which NAD or NADP is finally converted to a reduced form such as NADH or NADPH by a dehydrogenase reaction, and a dehydrogenase or a substrate for the dehydrogenase is measured based on an increase in the absorbance in an ultraviolet wavelength region (usually, 340 nm), which occurs as a result of such conversion. However, in this case, since it is measured in an ultraviolet region, convenient glass or plastic cannot be used for a measurement cell, and it is necessary to use expensive quartz. In addition, it is also necessary to use an ultraviolet light source and a detector in an analytical apparatus, and therefore, the analytical apparatus has been liable to have a large size and be expensive. As a result, the technique cannot be applied to apparatuses that use a visible spectrophotometer, with which measurement can be made even in the field of dry chemistry or at small hospitals. Therefore, in order to enable the technique to be used in such occasions, there is a demand for a method of measuring a dehydrogenase or a substrate for the dehydrogenase as described above in the visible region.

On the other hand, Patent Literature 1 suggests a method of performing an enzymatic cycling reaction by using the combination of thio-NAD or the thio-NADP and NADH or NADPH as a coenzyme when glucose-6-phosphate is allowed to react with glucose-6-phosphate dehydrogenase, and measuring glucose-6-phosphate using thio-NADH or thio-NADPH, which are both reduced form enzymes produced by the enzymatic cycling reaction, based on the absorbance obtained in the visible region near 400 nm. However, this method uses an enzymatic cycling reaction, and cannot be necessarily said to be a convenient measurement method. Furthermore, the method cannot be said to be a measurement method suitable for measuring objects of measurement at high concentrations.

### Citation List

### Patent Literature

Patent Literature 1: JP 04-335898 A

### Summary of Invention

### Technical Problem

It is an object of the present invention to provide a method which enables measurement based on the absorbance obtained in the visible region, can be applied to apparatuses that use a visible spectrophotometer and can make measurements even in the field of dry chemistry or at small hospitals, and is capable of accurately measuring a dehydrogenase or a substrate for the dehydrogenase, that is present in a sample at a high concentration.

### Solution to Problem

The present inventors paid attention to the fact that, for example, as described in Patent Literature 1, thio-NAD or thio-NADP, which are both coenzymes used to measure a dehydrogenase or a substrate for the dehydrogenase at a low concentration by an enzymatic cycling method, are able to alter the absorbance in the visible region through an enzymatic reaction, and thus the inventors conducted an investigation on a method for measuring an object of measurement that is present in a sample at a high concentration, using the coenzymes. As a result, it was found that when thio-NAD or thio-NADP is used as a coenzyme to measure an object of measurement at a high concentration, linearity is not easily maintained in a high concentration region, and in addition, the blank value may also be high, so that the object of measurement cannot be measured accurately.

The inventors made an attempt to measure a dehydrogenase or a substrate for the dehydrogenase in a sample by subjecting a certain kind of substance in the sample to an enzymatic reaction using NAD as a coenzyme so as to remove the substance using a certain enzyme, subsequently allowing a dehydrogenase, a substrate for the dehydrogenase and thio-NAD to react using the sample from which the substance has been removed, and then measuring the absorbance in the visible region. However, the inventors surprisingly discovered that when the absorbance in the visible region is measured using, as the sample, a simple constitution in which a dehydrogenase, a substrate for the dehydrogenase, thio-NAD and NAD are co-present, irrespective of the order of addition of NAD and thio-NAD, the reaction blank value decreases, and the gradient of the calibration curve is made low, so that the dehydrogenase or the substrate for the dehydrogenase in the sample can be accurately measured. As a result, it was found that when such a constitution is employed, the problems described above are addressed, and a dehydrogenase or a substrate for the dehydrogenase can be accurately measured even in a high concentration region.

Therefore, in regard to a method for measuring a dehydrogenase or a substrate for the dehydrogenase contained in a sample using the absorbance in the visible region, when thio-NAD or thio-NADP as well as NAD or NADP are both used as coenzymes, the object of measurement can maintain linearity even in a high concentration region, and meanwhile, the blank value of the calibration curve can be kept low. As a result, the inventors found that there can be provided a method for measuring a dehydrogenase or a substrate for the dehydrogenase simply and accurately in a broad region of the concentration of an object of measurement, using a convenient visible spectrophotometer, and thereby completed the present invention.

Therefore, the present invention relates to a measurement method of performing a reaction using a dehydrogenase, a substrate for the dehydrogenase, and coenzymes, and thereby measuring the dehydrogenase or the substrate for the dehydrogenase in a sample, in which the dehydrogenase or the substrate for the dehydrogenase in the sample is measured by performing the reaction using the combination of thio-NAD with NAD or thio-NADP with NADP as the coenzymes, and measuring an increase in the absorbance caused by the thio-NADH or thio-NADPH produced from the reaction.

Furthermore, the present invention relates to a measurement method in which a particular substance in a sample is used in an enzymatic reaction to produce a dehydrogenase or a substrate for the dehydrogenase as an intermediate or final product, the dehydrogenase or the substrate for the dehydrogenase is subjected to the measurement method described above, and the particular substance in the sample is measured based on the amount of the dehydrogenase or the substrate for the dehydrogenase that has been produced as an intermediate or a final product.

The present invention also relates to use of a measurement kit for measuring a dehydrogenase or a substrate for the dehydrogenase.

Furthermore, the present invention relates to use of a measurement kit for measuring a particular substance.

### Advantageous Effects of Invention

According to the present invention, even though a dehydrogenase or a substrate for the dehydrogenase contained in a sample is measured based on the absorbance obtainable in the visible region, the object of measurement can maintain linearity even in a high concentration region by using the combination of thio-NAD with NAD or thio-NADP with NADP as coenzymes, and the blank value of the calibration curve can be kept low. As a result, measurement can be made simply and accurately in a broad region of the concentration of the object of measurement, using a convenient visible spectrophotometer.

### Brief Description of Drawings

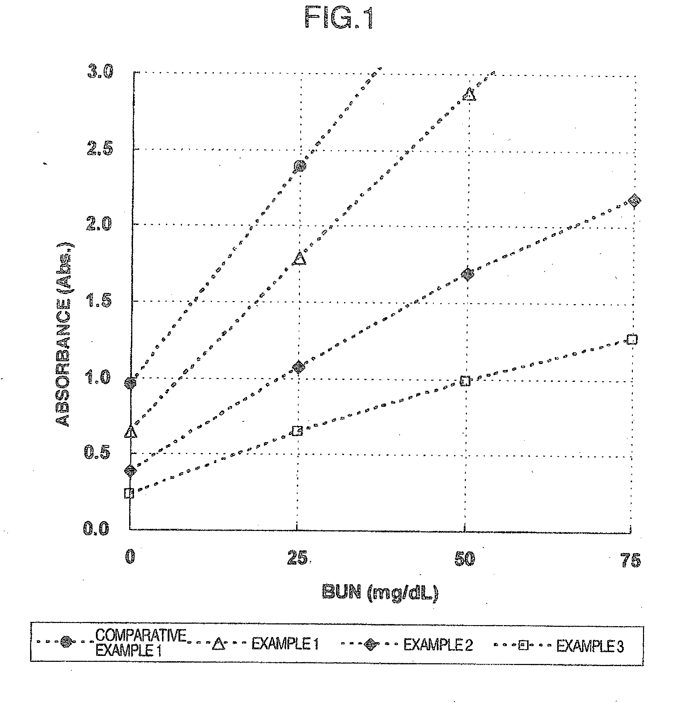

[Fig. 1] Fig. 1 is a diagram illustrating the results for the measurement of urea nitrogen by the methods of Examples 1, 2 and 3, and Comparative Example 1.

### Description of Embodiments

As the sample used in the present invention, a biological sample such as blood serum, blood plasma or urine, or a model sample thereof is preferred.

The object of measurement of the present invention is a dehydrogenase or a substrate for the dehydrogenase in a sample, or is a particular substance in a sample, which is capable of producing a dehydrogenase or a substrate for the dehydrogenase as an intermediate or a final product through an enzymatic reaction.

According to the present invention, when a dehydrogenase enzyme reaction is performed, the reaction is carried out by allowing thio-NAD or thio-NADP as well as NAD or NADP to be present as coenzymes. A combination of thio-NAD and NAD, or a combination of thio-NADP and NADP is used.

Hereinafter, thio-NAD or thio-NADP may be abbreviated to thio-NAD(P). Also, NAD or NADP may be abbreviated to NAD(P).

According to the present invention, thio-NAD means thionicotinamide adenine dinucleotide, and thio-NADP means thionicotinamide adenine dinucleotide phosphate. Furthermore, NAD means nicotinamide adenine dinucleotide, and NADP means nicotinamide adenine dinucleotide phosphate.

According to the present specification, thio-NADH, thio-NADPH, NADH and NADPH mean reduced forms of the coenzymes, that is, the reduced forms of thio-NAD, thio-NADP, NAD and NADP, respectively. Hereinafter, thio-NADH or thio-NADPH may be abbreviated to thio-NAD(P)H. Furthermore, NADH or NADPH may be abbreviated to NAD(P)H.

According to the present invention, there are no particular limitations on the amount of NAD(P) used, but the amount is preferably 0.5-fold moles or more, more preferably 1.5- to 30-fold moles, and most preferably 3.0- to 20-fold moles, relative to the amount of thio-NAD(P) used. If the amount of NAD(P) is too small, it may be difficult to maintain the linearity of the calibration curve, and the decrease in the blank value may be insufficient. On the other hand, if the amount of NAD(P) is too large, the gradient of the straight line of the calibration curve may be small, and it may be impossible to measure the object of measurement accurately.

In the present invention, a dehydrogenase reaction is carried out using both thio-NAD(P) and NAD(P) as coenzymes, and an increase in the absorbance caused by the thio-NAD(P)H produced from the reaction is measured. The wavelength for the measurement of absorbance is preferably in the range of 395 to 415 nm, and since the measurement can be made in the visible region, the measurement can be made using a visible spectrophotometer that is simple and easy to operate, as the analytic apparatus to be used.

According to the present invention, there are no particular limitations on the dehydrogenase or the substrate for the dehydrogenase, as long as the dehydrogenase or the substrate for the dehydrogenase is, for example, a dehydrogenase or a substrate for the dehydrogenase which can be measured by a dehydrogenase enzyme reaction using NAD(P) or thio-NAD(P) as a coenzyme that is currently used as an object of measurement in the industry of clinical laboratory tests. In this case, the dehydrogenase or the substrate for the dehydrogenase may be a dehydrogenase or a substrate for the dehydrogenase that is initially present in the sample. Further, the dehydrogenase or the substrate for the dehydrogenase may also be a dehydrogenase or a substrate for the dehydrogenase that has been produced as an intermediate or a final product when a particular substance is subjected to an enzymatic reaction in order to measure the particular substance in a sample. In this case, consequently, the particular substance can be measured, depending on the dehydrogenase or the substrate for the dehydrogenase produced from the enzymatic reaction.

Examples of a combination of a dehydrogenase and a substrate that may be used in such a method include a lactate dehydrogenase and lactic acid, a glutamate dehydrogenase and glutamic acid, an alcohol dehydrogenase and an alcohol, a glycerol-3-phosphate dehydrogenase and glycerol-3-phosphate, and a glucose-6-phosphate dehydrogenase and glucose-6-phosphate. In this case, in order to measure any one of the members of such a combination, the other member can be used as a reagent for carrying out a dehydrogenase enzyme reaction, together with coenzymes. According to the present invention, an object of measurement that is present in a sample at a high concentration is suitable.

In the case of using, as the dehydrogenase or the substrate for the dehydrogenase, a dehydrogenase or a substrate for the dehydrogenase that has been produced as an intermediate or a final product when a particular substance in a sample is subjected to an enzymatic reaction so as to measure the particular substance in the sample, an example of using a glucose-6-phosphate dehydrogenase as the dehydrogenase, and glucose-6-phosphate as the substrate for the dehydrogenase. In that occasion, for example, measurement can be made using urea (conventionally, urea may also be determined in terms of urea nitrogen) as a particular substance. In this case, for example, when urea in a sample is subjected to the action of ATP, magnesium ion, potassium ion, hydrogen carbonate ion, and urea amidolyase, which are provided by a reagent, in a first enzymatic reaction to produce ADP; subsequently, as a second enzymatic reaction, this ADP is subjected to the action of a hexokinase derived from a reagent to produce glucose-6-phosphate; and then the glucose-6-phosphate (substrate for the dehydrogenase) thus produced is subjected to the action of a glucose-6-phosphate dehydrogenase (dehydrogenase) as well as thio-NAD(P) and NAD(P) to induce a dehydrogenase enzyme reaction, glucose-6-phosphate (substrate for the dehydrogenase) thus produced can be measured from an increase in the absorbance, which is dependent on the amount of thio-NAD(P)H produced from the reaction. As a result, based on this procedure, urea can be measured as the particular substance in the sample. At this time, an ADP-dependent hexokinase is preferred as the hexokinase.

This example can be represented by the following reaction schemes.

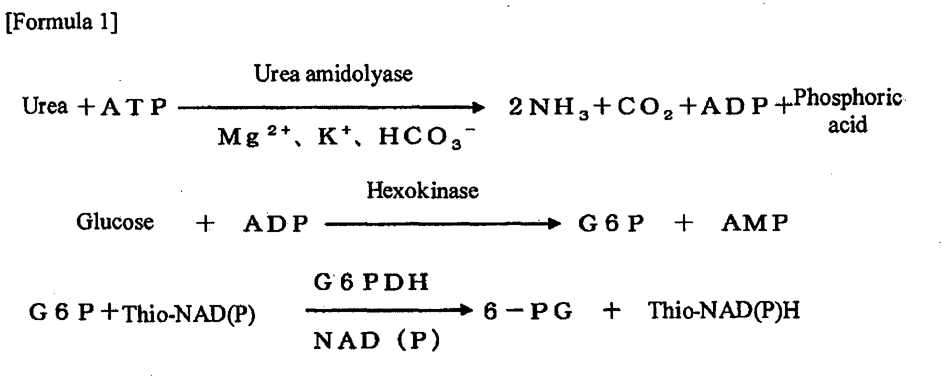

According to the present invention, when a dehydrogenase or a substrate for the dehydrogenase is measured as the object of measurement, for example, a composition containing, as an essential component, the dehydrogenase or the substrate for the dehydrogenase, and additionally containing, if necessary, other components such as a buffering agent and a surfactant, can be used as a first reagent, and a composition containing thio-NAD(P) and NAD(P) as essential components, and additionally containing other components such as a buffering agent and a surfactant can be used as a second reagent.

In this case, when glucose-6-phosphate is measured as a specific object of measurement, for example, the first reagent contains, as an essential component, glucose-6-5 phosphate dehydrogenase as the dehydrogenase, and the second reagent contains thio-NAD(P) and NAD(P) as essential components. In addition to these, the first reagent and the second reagent can contain other components such as a buffering agent and a surfactant.

When a dehydrogenase or a substrate for the dehydrogenase is measured as the object of measurement using such a first reagent and such a second reagent, for example, a 10 sample and the first reagent are mixed, and the mixture is left to stand for 0 to 15 minutes. Subsequently, the resulting liquid mixture and the second reagent are mixed, and thereby, an enzymatic reaction is carried out for 0.5 to 15 minutes. The absorbance of the reaction liquid thus obtained is measured at 405 nm with a visible spectrophotometer. When the measured value is compared with a calibration curve that has been prepared in advance, the concentration 15 of the object of measurement in the sample can be measured. Furthermore, in regard to the mixing of the sample and the reagents in this case, first, the first reagent and the second reagent are mixed to prepare a single measuring reagent, and the measuring reagent and the sample can be mixed. Furthermore, the enzymatic reaction can be carried out also by subjecting the resulting mixture to react for 0.5 to 15 minutes.

According to the present invention, when a particular substance is measured as the object of measurement, for example, the first reagent can be allowed to contain, as essential components, enzymatic reaction-including components for producing a substrate for a dehydrogenase from the particular substance by an enzymatic reaction, and the dehydrogenase, 25 and can be allowed to additionally contain other components such as a buffering agent and a surfactant, if necessary. The second reagent can be allowed to contain thio-NAD(P) and NAD(P) as essential components, and to additionally contain other components such as a buffering agent and a surfactant.

In this case, when urea as the particular substance is measured as a specific object 30 of measurement, for example, a composition containing first enzymatic reaction-inducing components for producing ADP from urea (that is, urea amidolyase, ATP, Mg²⁺, K⁺, and HCO₃⁻), and second enzymatic reaction-inducing components for producing glucose-6-phosphate (that is, substrate for the dehydrogenase) from ADP (that is, glucose and hexokinase), as components that derive glucose-6-phosphate (that is, substrate for the dehydrogenase) from urea (that is, particular substance) through an enzymatic reaction; and glucose-6-phosphate dehydrogenase as a dehydrogenase for the reagent components, can be used as the first reagent. The second reagent contains both thio-NAD(P) and NAD(P) as essential components. In addition to those, the first reagent and the second reagent can contain other components such as a buffering agent and a surfactant.

When the particular substance is measured as the object of measurement using such a first reagent and such a second reagent, for example, the sample and the first reagent are mixed, and the mixture is left to stand for 0 to 15 minutes. Subsequently, the resulting liquid mixture and the second reagent are mixed, and thereby, an enzymatic reaction is carried out for 0.5 to 15 minutes. The absorbance of the reaction liquid thus obtained is measured at 405 nm using a visible spectrophotometer. When the measured value is compared with a calibration curve that has been prepared in advance, the concentration of the object of measurement in the sample can be measured. Furthermore, in regard to the mixing of the sample and the reagents in this case, first, the first reagent and the second reagent are mixed to prepare a single measuring reagent, and the measuring reagent and the sample can be mixed. Furthermore, the enzymatic reaction can be carried out also by subjecting the resulting mixture to react for 0.5 to 15 minutes.

As is obvious from the descriptions given above, according to the present invention, use can be made of a measurement kit for measuring a dehydrogenase or a substrate for the dehydrogenase, which contains, as an essential component, the dehydrogenase or the substrate for the dehydrogenase, and contains, as essential components, the combination of thio-NAD with NAD or thio-NADP with NADP as coenzymes.

In this case, for example, in order to measure a dehydrogenase or a substrate for the dehydrogenase, use can be made of a measurement kit for measuring a dehydrogenase or a substrate for the dehydrogenase, which includes a first reagent containing, as an essential component, the dehydrogenase or the substrate for the dehydrogenase, and a second reagent containing the combination of thio-NAD with NAD or thio-NADP with NADP as essential components.

Furthermore, according to the present invention, in order to measure a particular substance, use can be made of a measurement kit for measuring a particular substance, which contains, as essential components, enzymatic reaction-including components for producing a substrate for a dehydrogenase from the particular substance through an enzymatic reaction, and the dehydrogenase, and also contains, as essential components, the combination of thio-NAD with NAD or thio-NADP with NADP as coenzymes.

In this case, use can be made of, for example, a measurement kit for measuring a particular substance, which includes a first reagent containing, as essential components, enzymatic reaaction-including components for producing a substrate for a dehydrogenase from the particular substance through an enzymatic reaction, and the dehydrogenase, and a second reagent containing the combination of thio-NAD with NAD or thio-NADP with NADP as essential components.

In the first and second reagents in these measurement kits, other components such as a buffering agent and a surfactant can be optionally incorporated, as described above.

### Examples

Hereinafter, the present invention will be described in more detail by way of Examples and a Comparative Example, but the present invention is not intended to be limited to these examples.

### Examples 1, 2 and 3, and Comparative Example 1

### Measurement of urea nitrogen using visible region analyzer

In order to measure urea nitrogen as the particular substance in a sample, as a first enzymatic reaction, urea nitrogen in the sample was subjected to the action of ATP, magnesium ion, potassium ion, hydrogen carbonate ion, and urea amidolyase, which were provided by a reagent, to produce ADP. Subsequently, as a second enzymatic reaction, this ADP was subjected to the action of a hexokinase from a reagent to produce glucose-6-phosphate. Subsequently, the glucose-6-phosphate (substrate for the dehydrogenase) thus produced was subjected to the action of glucose-6-phosphate dehydrogenase (dehydrogenase) as well as thio-NAD and NAD, and glucose-6-phosphate (substrate for the dehydrogenase) thus produced was measured from an increase in the absorbance, which is dependent on the amount of thio-NADH produced. Thereby, the urea nitrogen as the particular substance in a sample was measured. Furthermore, as a comparison control, the same measurement was carried out using thio-NAD alone.

### 1. Method

In regard to the urea nitrogen measurement systems using urea amidolyase, a hexokinase and glucose-6-phosphate dehydrogenase, measurement systems in which thio-NAD and NAD were mixed as coenzymes (Examples 1, 2 and 3), and a measurement system in which thio-NAD was used alone (Comparative Example 1) were compared. In this case, the urea was quantified as the amount of urea nitrogen, The sample and the reagents were described below

### Sample:

A urea solution was prepared to a concentration of 75 mg/dL in terms of urea nitrogen, and the urea solution was appropriately diluted with physiological saline to various concentrations.

### First reagent:

In the first reagent, the amount of each component was adjusted so as to have the following concentration,

| | |
|---|---|
| 20 mM | Tris (tris(hydroxymethyl)aminomethane) pH 9.0 |
| 10 mM | D-glucose |
| 10 mM | Magnesium acetate tetrahydrate |
| 10 mM | Potassium hydrogen carbonate |
| 10 mM | ATP.2Na (disodium adenosine triphosphate) |
| 1% | Surfactant |
| 6 KU/L | Hexokinase |
| 2 KU/L | Urea amidolyase |
| 2 KU/L | Glucose-6-phosphate dehydrogenase |

### Second reagent:

In the second reagent, the amounts of Tris and the surfactant were adjusted so as to have the following respective concentrations.

| | |
|---|---|
| 200 mM | Tris |
| | pH 7.2 |
| x mM | Thio-NAD |
| y mM | NAD |
| 1% | Surfactant |

The addition amounts of thio-NAD and NAD in the second reagent are presented in Table 1. Furthermore, in Comparative Example 1, NAD was excluded from the composition of the above second reagent composition, and thio-NAD was used alone.

### [Table 1]

**Table 1**

| | Comparative Example 1 | Example 1 | Example 2 | Example 3 |
|---|---|---|---|---|
| Mixing ratio of thio-NAD:NAD | 1:0 | 1 : 1 | 1 : 4 | 1 : 9 |
| X mM (thio-NAD) | 4 | 2 | 0.8 | 0.4 |
| ymM (NAD) | 0 | 2 | 3.2 | 3.6 |

### Measurement method:

The measurement was carried out as described below 3.0 µL of the sample and 100 µL of the first reagent were mixed for 5 minutes at 37°C using a visible region analyzer. Subsequently, 100 µL of the second reagent was added to the mixture, and the resulting mixture was allowed to react for 5 minutes at the same temperature. The absorbance after the color development reaction was measured at a principal wavelength of 405 nm and a sub-wavelength of 505 nm, by a one-point end method for the relevant analyzer model.

### 2. Results

The results thus obtained are presented in Fig. 1. From the results shown in Fig. 1, it was found that when NAD was incorporated in addition to thio-NAD, the measurement blank value was low, and the gradient also decreased. In the compositions using thio-NAD and NAD in mixture, measurement could be achieved up to 50 mg/dL in Example 1, and up to 75 mg/dL in Examples 2 and 3.

On the other hand, in the composition which used thio-NAD alone as Comparative Example 1, the linearity exceeded beyond the detectable absorbance range at a urea nitrogen concentration of 25 mg/dL or higher, and therefore, measurement could not be achieved.

### Industrial Applicability

According to the present invention, when a dehydrogenase or a substrate for the dehydrogenase contained in a sample is measured by means of an enzymatic reaction, the dehydrogenase or the substrate for the dehydrogenase can be measured based on the absorbance in the visible region, by using thio-NAD or thio-NADP as well as NAD or NADP as coenzymes. Thereby, linearity can be maintained even in a high concentration region of the object of measurement, and the blank value of the calibration curve can also be kept low. As a result, measurement can be made simply and accurately in a broad region of the concentration of the object of measurement, using a convenient visible spectrophotometer.

## Claims

1. A measurement method for measuring a dehydrogenase or a substrate for the dehydrogenase in a sample by performing a reaction using the dehydrogenase, the substrate for the dehydrogenase and coenzymes, the measurement method comprising performing the reaction using the combination of thio-NAD with NAD or thio-NADP with NADP as coenzymes; and measuring an increase in the absorbance caused by the thio-NADH or thio-NADPH produced from the reaction.

2. The measurement method according to claim 1, wherein the NAD or NADP is used in an amount of 0.5 times or more the mole number of thio-NAD or thio-NADP.

3. A measurement method for measuring a particular substance in a sample, the method comprising producing a dehydrogenase or a substrate for the dehydrogenase as an intermediate or a final product by subjecting a particular substance in a sample to an enzymatic reaction; subjecting the dehydrogenase or the substrate for the dehydrogenase to the measurement method according to claim 1 or 2; and measuring the particular substance based on the amount of the dehydrogenase or the substrate for the dehydrogenase produced as an intermediate or a final product.

4. The measurement method according to any one of claims 1 to 3, wherein the dehydrogenase is glucose-6-phosphate dehydrogenase, and the substrate for the dehydrogenase is glucose-6-phosphate.

5. The measurement method according to claim 4, wherein the enzymatic reaction comprises plural enzymatic reactions.

6. The measurement method according to claim 5, wherein the dehydrogenase is glucose-6-phosphate dehydrogenase; the substrate for the dehydrogenase is glucose-6-phosphate; the particular substance is urea; the first enzymatic reaction is a reaction of subjecting urea in the sample to the action of ATP, magnesium ion, potassium ion, hydrogen carbonate ion, and urea amidolyase, which are provided by a reagent, to produce ADP as an intermediate; and the second enzymatic reaction is a reaction of subjecting the ADP to the action of a hexokinase from a reagent, to produce glucose-6-phosphate.

7. Use of a measurement kit for measuring a dehydrogenase, wherein the measurement kit comprises a first reagent containing, as an essential component, a substrate for the dehydrogenase, and a second reagent containing, as essential components, the combination of thio-NAD with NAD or thio-NADP with NADP.

8. Use of a measurement kit for measuring a substrate for a dehydrogenase, wherein the measurement kit comprises a first reagent containing, as an essential component, the dehydrogenase, and a second reagent containing, as essential components, the combination of thio-NAD with NAD or thio-NADP with NADP.

9. Use of a measurement kit for measuring a particular substance, wherein the measurement kit comprises:
a first reagent containing, as essential components, enzymatic reaction-inducing components for producing a substrate for a dehydrogenase from the particular substance through an enzymatic reaction, and the dehydrogenase; and
a second reagent containing, as essential components, the combination of thio-NAD with NAD or thio-NADP with NADP.

## Patentansprüche

1. Messverfahren zum Messen einer Dehydrogenase oder eines Substrats für die Dehydrogenase in einer Probe durch Durchführen einer Reaktion unter Verwendung der Dehydrogenase, des Substrats für die Dehydrogenase und von Coenzymen, wobei das Messverfahren die Durchführung der Reaktion unter Verwendung der Kombination von Thio-NAD mit NAD oder Thio-NADP mit NADP als Coenzyme umfasst; und Messen einer Zunahme in der Absorption, die durch das von der Reaktion erzeugte Thio-NADH oder Thio-NADPH verursacht wird.

2. Messverfahren gemäß Anspruch 1, bei dem das NAD oder NADP in einer Menge des 0,5-fachen oder mehr der Molzahl von Thio-NAD oder Thio-NADP verwendet wird.

3. Messverfahren zum Messen einer bestimmten Substanz in einer Probe, wobei das Verfahren umfasst, dass eine Dehydrogenase oder ein Substrat für die Dehydrogenase als ein Zwischenprodukt oder ein Endprodukt hergestellt wird, indem eine bestimmte Substanz in einer Probe einer enzymatischen Reaktion unterzogen wird; die Dehydrogenase oder das Substrat für die Dehydrogenase dem Messverfahren nach Anspruch 1 oder 2 unterzogen wird; und die bestimmte Substanz auf Basis der Menge der Dehydrogenase oder des Substrats für die Dehydrogenase, die als ein Zwischenprodukt oder ein Endprodukt erzeugt wird, gemessen wird.

4. Messverfahren gemäß mindestens einem der Ansprüche 1 bis 3, bei dem die Dehydrogenase Glucose-6-phosphat-Dehydrogenase ist und das Substrat für die Dehydrogenase Glucose-6-phosphat ist.

5. Messverfahren gemäß Anspruch 4, bei dem die enzymatische Reaktion mehrere enzymatische Reaktionen umfasst.

6. Messverfahren gemäß Anspruch 5, bei dem die Dehydrogenase Glucose-6-phosphat-Dehydrogenase ist; das Substrat für die Dehydrogenase Glucose-6-phosphat ist; die bestimmte Substanz Harnstoff ist; die erste enzymatische Reaktion eine Reaktion ist, bei der Harnstoff in der Probe der Wirkung von ATP, Magnesiumion, Kaliumion, Hydrogencarbonation und Harnstoff-Amidolyase, die durch ein Reagenz bereitgestellt werden, ausgesetzt wird und so ADP als ein Zwischenprodukt erzeugt wird; und die zweite enzymatische Reaktion eine Reaktion ist, bei der das ADP der Wirkung einer Hexokinase aus einem Reagenz augesetzt wird und so Glucose-6-phosphat erzeugt wird.

7. Verwendung eines Messkits zum Messen einer Dehydrogenase, wobei das Messkit ein erstes Reagenz, das als essentiellen Bestandteil ein Substrat für die Dehydrogenase enthält, und ein zweites Reagenz, das als essentielle Bestandteile die Kombination von Thio-NAD mit NAD oder Thio-NADP mit NADP enthält, umfasst.

8. Verwendung eines Messkits zum Messen eines Substrats für eine Dehydrogenase, wobei das Messkit ein erstes Reagenz, das als essentiellen Bestandteil die Dehydrogenase enthält, und ein zweites Reagenz, das als essentielle Bestandteile die Kombination von Thio-NAD mit NAD oder Thio-NADP mit NADP enthält, umfasst.

9. Verwendung eines Messkits zum Messen einer bestimmten Substanz, wobei das Messkit folgendes umfasst:
ein erstes Reagenz, das als essentielle Bestandteile enzymatische Reaktion-induzierende Bestandteile zur Erzeugung eines Substrats für eine Dehydrogenase aus der bestimmten Substanz durch eine enzymatische Reaktion und die Dehydrogenase enthält; und
ein zweites Reagenz, das als essentielle Bestandteile die Kombination von Thio-NAD mit NAD oder Thio-NADP mit NADP enthält.

## Revendications

1. Procédé de mesure destiné à mesurer une déshydrogénase ou un substrat de la déshydrogénase dans un échantillon en exécutant une réaction qui utilise la déshydrogénase, le substrat de la déshydrogénase et des coenzymes, le procédé de mesure comprenant les étapes consistant à : exécuter la réaction en utilisant une association de thio - NAD et de NAD ou de thio - NADP et de NADP en tant que coenzymes ; et mesurer une augmentation de l'absorbance provoquée par le thio - NADH ou par le thio - NADPH produite à partir de la réaction.

2. Procédé de mesure selon la revendication 1, dans lequel le NAD ou le NADP est utilisé en une quantité égale à 0,5 fois ou plus le nombre molaire de thio - NADP ou de thio - NADP.

3. Procédé de mesure destiné à mesurer une substance particulière dans un échantillon, le procédé comprenant les étapes consistant à : produire une déshydrogénase ou un substrat de la déshydrogénase en tant que produit intermédiaire ou final en soumettant une substance particulière dans un échantillon à une réaction enzymatique ; soumettre la déshydrogénase ou le substrat de la déshydrogénase au procédé de mesure selon la revendication 1 ou la revendication 2 ; et mesurer la substance particulière sur la base de la quantité de déshydrogénase ou de substrat de la déshydrogénase produite en tant que produit intermédiaire ou final.

4. Procédé de mesure selon l'une quelconque des revendications 1 à 3, dans lequel la déshydrogénase est une déshydrogénase de glucose - 6 - phosphate, et le substrat de la déshydrogénase est le glucose - 6 - phosphate.

5. Procédé de mesure selon la revendication 4, dans lequel la réaction enzymatique comprend plusieurs réactions enzymatiques.

6. Procédé de mesure selon la revendication 5, dans lequel la déshydrogénase est une déshydrogénase de glucose - 6 - phosphate ; le substrat de la déshydrogénase est le glucose - 6 - phosphate ; la substance particulière est l'urée ; la première réaction enzymatique est une réaction consistant à soumettre l'urée présente dans l'échantillon à l'action de l'ATP, d'un ion de magnésium, d'un ion de potassium, d'un ion de carbonate d'hydrogène, et d'une amidolyase d'urée, qui sont fournis par un réactif, de façon à produire l'ADP en tant qu'intermédiaire ; et la seconde réaction enzymatique est une réaction consistant à soumettre l'ADP à l'action d'une hexokinase à partir d'un réactif, de façon à produire le glucose - 6 - phosphate.

7. Utilisation d'un kit de mesure destiné à mesurer une déshydrogénase, dans laquelle le kit de mesure comprend un premier réactif qui contient, en tant que composant essentiel, un substrat de la déshydrogénase, et un second réactif qui contient, en tant que composants essentiels, l'association de thio - NAD et de NAD ou de thio - NADP et de NADP.

8. Utilisation d'un kit de mesure destiné à mesurer un substrat d'une déshydrogénase, dans laquelle le kit de mesure comprend un premier réactif qui contient, en tant que composant essentiel, la déshydrogénase, et un second réactif qui contient, en tant que composants essentiels, l'association de thio - NAD et de NAD ou de thio - NADP et de NADP.

9. Utilisation d'un kit de mesure destiné à mesurer une substance particulière, dans laquelle le kit de mesure comprend :
un premier réactif qui contient, en tant que composants essentiels, des composants qui induisent une réaction enzymatiques destinée à produire un substrat d'une déshydrogénase à partir de la substance particulière par l'intermédiaire d'une réaction enzymatique, et la déshydrogénase ; et
un second réactif qui contient, en tant que composants essentiels, l'association de thio - NAD et de NAD ou de thio - NADP et de NADP.
